# EUROPEAN PATENT SPECIFICATION

(11) **EP 2 293 830 B1**
(45) Date of publication and mention of the grant of the patent: **08.08.2012**
(21) Application number: 09757864.5
(22) Date of filing: 04.06.2009
(51) Int. Cl.: A61M 1/36

(54) **CATHETER FLUSH AND FILL UNIT**
EINHEIT ZUM DURCHSPÜLEN UND FÜLLEN EINES KATHETERS
UNITÉ DE RINÇAGE ET DE REMPLISSAGE DE CATHÉTER

(30) Priority: 05.06.2008 IT TO20080433
(43) Date of publication of application: 16.03.2011
(73) Proprietor: Haemotronic S.p.A., Mirandola (MO) (IT)
(72) Inventor: GOLDONI, Marco, 46020 Carbonara di Po (IT); RAVIZZA, Alice, 46020 Carbonara di Po (IT)
(74) Representative: Mola, Edoardo
(86) International application number: PCT/IB2009/005847
(87) International publication number: WO 2009/147510

(56) References cited:
- EP-A- 0 075 039
- WO-A-02/066099
- US-A- 5 334 315

## Description

### TECHNICAL FIELD

The present invention relates to a catheter flush and fill unit, e.g. for catheters inserted into blood vessels or the peritoneal cavity.

### BACKGROUND ART

A catheter is normally used for direct access to a patient's circulatory system or other parts of the body, such as the peritoneal cavity, and permits direct access from the outside to the system into which it is inserted. To safeguard the health of the patient, it is essential to ensure a fluidtight, hygienic connection between the catheter and other medical devices connected to it.

When the catheter is not connected to any other device, the end outside the patient's body is closed hermetically by clamps and then by screw caps.

Connecting the catheter to any medical device connectable to it comprises first opening the free end of the catheter, aspirating any clots and filling the catheter tube with sterile liquid using one or more syringes, and checking the patency of the catheter tube.

If the catheter tube is clogged with stubborn fibrous deposits, such as clots, etc., a fibrinolytic drug is injected into the tube and/or a mechanical process of aspiration and injection of sterile liquid into the tube using a syringe is performed to flush out the occlusions by pressure exerted manually on the plunger of the syringe.

Preliminary flushing of catheter tubes may produce fairly large clots.

Because it involves connecting, disconnecting, and reconnecting one or more syringes, flushing is performed in an open environment, and part of the fluid withdrawn from the catheter and consisting of a mixture of the injected sterile liquid and biological fluids is discharged into an open container for visual inspection by the operator.

The above process is performed one or more times, as required, to clear the catheter with the pressure exerted by the operator on the syringe.

The above process, which is currently performed whenever a catheter is connected to any external device, poses health risks to both the patient and the operator.

The patient, on the one hand, is exposed to the risk of infection on account of the number of connections and disconnections involved. Moreover, when the aspirated fluid is discharged by the operator into an open container for inspection, the catheter-side connection remains open, thus greatly increasing the risk of contamination of the tube.

The operator, on the other hand, is exposed to all the hazards, such as infection and contamination, entailed in working openly with biological fluids.

Some instruments are known for removing clots from the patient's circulatory system. These clots, unlike those for removal by the unit according to the present invention, adhere to the endothelium lining the blood vessel, as opposed to the inside of the catheter tube.

Clots inside blood vessels are removed mechanically, as described, for example, in US2005248953, in which a rotary probe is inserted into the occluded blood vessel.

US-A-5334315 discloses a unit according to the preamble of claim 1.

### DISCLOSURE OF INVENTION

It is an object of the present invention to provide a catheter flush and fill unit designed to eliminate open preliminary flushing of the catheter tube.

The object of the present invention is achieved by a catheter flush and fill unit designed to eliminate the aforementioned drawback as described in claim 1.

### BRIEF DESCRIPTION OF THE DRAWINGS

The present invention will be described with reference to the accompanying drawings, in which:
Figure 1 shows a plan view of a catheter flush and fill unit in accordance with the present invention;
Figure 2 shows a longitudinal section of a component part of the Figure 1 unit.

### BEST MODE FOR CARRYING OUT THE INVENTION

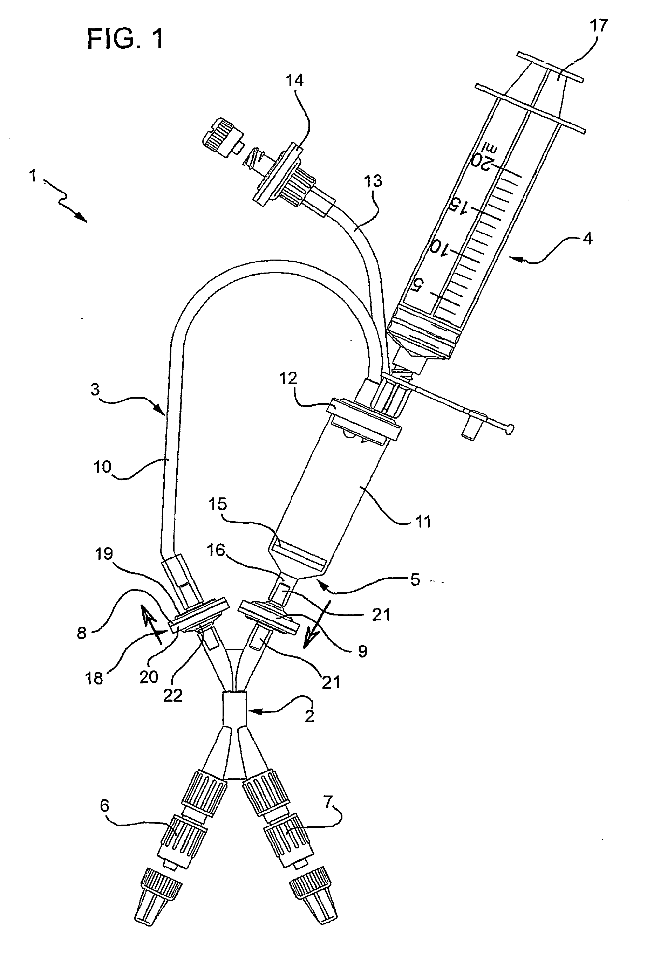

Number 1 in Figure 1 indicates as a whole a catheter flush and fill unit comprising a connection assembly 2 for connection to a catheter (not shown); a suction branch 3 connected to connection assembly 2; a syringe 4 downstream from suction branch 3; and a delivery branch 5 downstream from syringe 4 and connected to connection assembly 2. Connection assembly 2, suction and delivery branches 3 and 5, and syringe 4 are preferably connected unremovably.

Connection assembly 2 comprises a first port 6 and a second port 7 featuring, for example, "luer" connections; and a non-return valve 8 and non-return valve 9 connected fluidically parallel with respect to ports 6 and 7 to permit one-way flow in unit 1. Ports 6 and 7 are connected to a common compartment defined by the connection assembly, so as to be short-circuited.

Suction branch 3 comprises a conduit 10 connected to non-return valve 8, and delivery branch 5 comprises a reservoir 11 connected between conduit 10 and non-return valve 9. More specifically, reservoir 11 comprises an elongated cylindrical casing having an end portion 12 connected to conduit 10, to syringe 4, and to a conduit 13 defining a port 14 comprising a sterilizing filter for injecting physiological solution into unit 1, as explained in detail below. The cylindrical casing is transparent for visual blood inspection.

Unit 1 also comprises a haemocompatible filter 15 downstream from syringe 4 and designed to let through the corpuscle part of the blood without damage, but to retain larger solid or gelatinous bodies, such as clot or fibrous adhesion fragments. More specifically, haemocompatible filter 15 retains particles of over 25 microns in size, is preferably static, and is housed in an end portion 16, opposite end portion 12, of reservoir 11.

More specifically, haemocompatible filter 15 is fitted non-removably inside reservoir 11 to define, with the reservoir, with connection assembly 2, and with conduit 10, a disposable unit for circulating biological fluid in a closed, sterile environment.

Unit 1 is supplied in a sterile package, and all the connections to the outside, i.e. the connections of ports 6, 7 and 14, are closed by microporous caps by which to sterilize the inside of unit 1 by diffusion of appropriate gases.

For the same reason, syringe 4 is a safety syringe, i.e. in which plunger 17 is non-removable, and is movable between two limit positions defining the pumping volume and located at end portions 12 and 16 respectively.

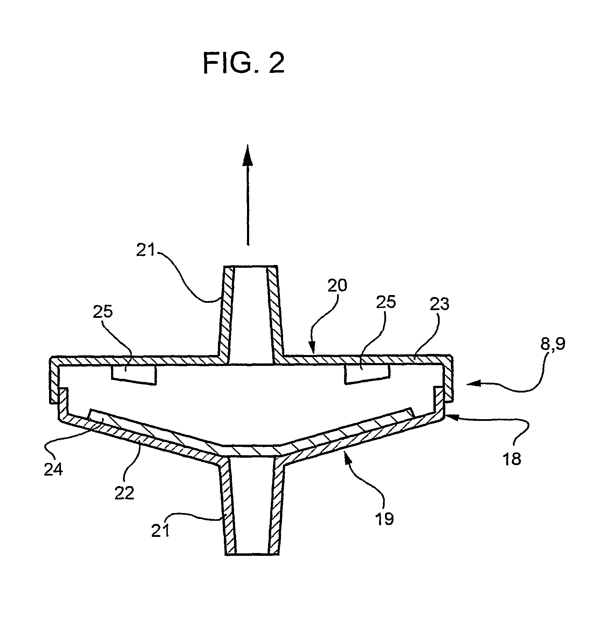

In a preferred embodiment of the present invention, non-return valves 8, 9 (Figure 2) each comprise an outer casing 18 having a first and second half-shell 19, 20 connected hermetically to each other and each comprising a tubular connecting portion 21. The upstream half-shell 19 has a flared wall 22 projecting from connecting portion 21, and the downstream half-shell 20 has a substantially flat wall 23 projecting from connecting portion 21. Each non-return valve 8, 9 also comprises a membrane 24 of flexible plastic material, e.g. a thin plate of PVC, mounted in floating manner inside outer casing 18.

The downstream half-shell 20 defines a number of axial projections 25, preferably not axially symmetrical, to limit movement of the membrane in the flow direction. In a preferred embodiment of the present invention, each projection is in the form of a right-angle trapezium, and is positioned so its sloping side slopes the same way as the concavity of the upstream half-shell 19.

In actual use, unit 1 is removed from a sterile package, and port 14 is connected to a syringe (not shown) of physiological solution, possibly containing an anticoagulant additive. At this start or priming stage, the content of the syringe completely fills every cavity of unit 1, and the operator makes sure no air is left inside; sterility is maintained by the sterilizing filter of port 14; and non-return valves 8, 9 are activated, i.e. are filled completely with fluid, and no air remains inside.

Ports 6 and 7 are then connected respectively to the vein and artery lines, leaving the clamp on the artery line closed.

At this point, the operator pulls plunger 17, and the blood from the catheter mixes with the physiological solution in unit 1 to form a liquid that flows through non-return valve 8 and along conduit 10 into reservoir 11. At this stage, non-return valve 8 is open, and non-return valve 9 closed.

The operator determines the patency of the catheter by observing the presence of blood in unit 1 and evaluating the manual pressure required to move plunger 17, and also checks for the presence of solid fragments inside reservoir 11.

At this point, the operator pushes plunger 17, the fluid flows through haemocompatible filter 15, which retains the clots but lets through the corpuscle part of the blood, and the operator determines the patency and flow rate of the catheter by evaluating the manual pressure and time taken to push the plunger right down.

The fluid from reservoir 11 flows through non-return valve 9 and, non-return valve 8 being closed, flows back, filtered, into the vascular system.

Once the clots are removed on the vein side, this is clamped; the artery port 7 is activated by means of the relative clamp; and pumping is repeated as long as necessary to clear the artery side.

The catheter flush and fill unit according to the present invention has the following advantages.

Non-return valves 8, 9 provide for one-way flow along unit 1, thus preventing clots from flowing back into the catheter when setting it up, and are easy to produce and assemble. Moreover, suction and delivery branches 3 and 5 define a closed biological fluid circuit, i.e. not exposed to external agents, thus greatly reducing the risk of infection.

The risk of infection is also greatly reduced by the system involving only one connection to the catheter. The transparent reservoir 11 enables visual inspection of the biological fluid to determine the presence of clots or corpuscles. Plunger 17 may be operated repeatedly, at the operator's discretion, without having to eliminate the biological liquid. At the end of each cycle, the content of reservoir 11 can be inspected visually by the operator, so the whole operation of determining the patency of the catheter is completed with only one connection to the catheter.

Moreover, ports 6, 7, 14 have "luer", i.e. standard, universal, connections.

"Luer" connections are safety connections, i.e. threaded as opposed to simply push-in types, and so safeguard against accidental disconnection which may result, for example, in blood loss, thus aggravating the patient's condition or conditioning the operator.

Should haemocompatible filter 15 clog when plunger 17 is fully pulled, unit 1 should be disconnected. Since reservoir 11 and syringe 4 have a total maximum volume of only 35ml, however, the amount of blood withdrawn from the patient is very small.

The component parts of unit 1 have a simple geometry, and the whole unit can be made of sterilizable medical polymer materials.

Clearly, changes may be made to the unit as described and illustrated herein without, however, departing from the scope of the present invention.

For example, connection assembly 2 may comprise only one port for connection to the catheter. In which case, the port is connected simultaneously to both the suction branch 3 and delivery branch 5, and is used to clear single-tube catheters.

Syringe 4 may be connected removably or non-removably between the suction and delivery branches.

Conduit 13 and port 14 are optional.

A withdrawal/injection access, i.e. and injection site, may be connected along conduit 10. That is, once flushing is completed, the unit may be left connected to the catheter and serve as an interface for withdrawals or injections. Moreover, once flushing is completed, the blood flowing along conduit 10 is clean and clot-free.

In a variation of the present invention, reservoir 11 is collapsible, and in particular deforms in a manner perceivable by touch and/or visibly when the pressure inside is below atmospheric pressure. This alerts the operator to severe clogging, e.g. caused by clots, along the flush unit fluid circuit and/or in the catheter, close to the connection to the flush unit. A sharp fall in pressure when using the flush unit may be transferred to the patient's blood vessels and cause injury, including even lacerated and broken blood vessels. Low pressure also damages the corpuscle part of the blood, in particular the red blood cells.

These effects are known, and are currently avoided by the skill of the operator, who usually measures aspiration time of blood when the plunger is pulled. The variation of the flush unit according to the present invention enables clogging to be detected, and injury caused by excessively low pressure to be avoided, more objectively and reliably.

Dialysis machines are also normally programmed to emit an alarm signal in the event of severe clogging of the catheter. For example, a dialysis machine comprises a pressure sensor, and the control system of the machine is set to a minimum pressure, below which an alarm signal is emitted. The minimum pressure may be -300 mmHg (relative to atmospheric pressure) and is at any rate below -250 mmHg.

To make the flush unit compatible with dialysis machines, reservoir 11 is designed to deform, in particular collapse, when the pressure inside reservoir 11, at the suction stage using syringe 4, is below -250 mmHg and more preferably below -300 mmHg. Collapsible reservoir 11 preferably also defines a handgrip, by which to grip the flush unit and so make it easier for the operator to determine when the minimum pressure is exceeded.

## Claims

1. A catheter flush and fill unit comprising a connector (2) for connection to a catheter; a non-return valve (8) connected fluidically to said connector (2); a suction branch (3) connected to said non-return valve (8); a filter (15) downstream from said suction branch (3) ; a delivery branch (5) downstream from said suction branch (3), and a further non-return valve (9) connecting said delivery branch (5) to said, connector (2); said delivery branch (5) comprising a reservoir (11) having a transparent lateral wall; and said filter (15) being inside said reservoir (11), the unit being **characterized in that** said further non-return valve (9) is downstream of said reservoir (11) so that fluid flows from said reservoir (11) through said further non-return valve (9)

2. A unit as claimed in Claim 1, **characterized in that** said filter (15) is not removable from said reservoir (15).

3. A unit as claimed in either one of the foregoing Claims, **characterized in that** at least one of said non-return valves (8, 9) comprises a floating flexible membrane.

4. A unit as claimed in any one of the foregoing Claims, **characterized by** comprising a pumping member (17) located in series between said suction branch (3) and said delivery branch (5).

5. A unit as claimed in any one of the foregoing Claims, **characterized in that** at least said connector (2), said suction branch (3), and said delivery branch (5) are connected non-removably.

6. A unit as claimed in any one of the foregoing Claims, **characterized by** being made of biomedical polymer materials.

7. A unit as claimed in any one of the foregoing Claims, **characterized by** comprising an inlet port (14) comprising a sterilizing filter and located in series between said suction branch (3) and said delivery branch (5).

8. A method of employing a unit as claimed in Claim 7, **characterized by** the operation of filling said unit completely with a liquid injected through said inlet port (14).

9. A flush unit as claimed in any one of Claims 1 to 7, **characterized by** comprising a collapsible member (11) collapsible in a manner perceivable by touch and/or visibly.

10. A flush unit as claimed in Claim 9, **characterized in that** said collapsible member (11) comprises said reservoir.

11. A flush unit as claimed in either one of Claims 9 and 10, **characterized in that** said collapsible member (11) collapses when the pressure inside is less than -250 mmHg.

## Patentansprüche

1. Katheterspül- und -fiilleinheit, umfassend einen Anschluss (2) zur Verbindung mit einem Katheter, ein Rückschlagventil (8), das mit dem Anschluss (2) strömungstechnisch verbunden ist; eine Saugverzweigung (3), die mit dem Rückschlagventil (8) verbunden ist; einen Filter (15), der der Saugverzweigung (3) nachgelagert ist; eine Zufiihrungsverzweigung (5), die der Saugverzweigung (3) nachgelagert ist, und ein weiteres Rückschlagventil (9), das die Zuführungsverzweigung (5) mit dem Anschluss (2) verbindet; wobei die Zuführungsverzweigung (5) einen Behälter (11) umfasst, der eine transparente seitliche Wand aufweist; und wobei der Filter (15) in dem Behälter (11) ist, wobei die Einheit **dadurch gekennzeichnet ist, dass** das weitere Rückschlagventil (9) dem Behälter (11) nachgelagert ist, so dass ein Fluid von dem Behälter (11) durch das weitere Rückschlagventil (9) fließt.

2. Einheit nach Anspruch 1, **dadurch gekennzeichnet, dass** der Filter (15) von dem Behälter (15) nicht entfernbar ist.

3. Einheit nach einem der vorstehenden Ansprüche, **dadurch gekennzeichnet, dass** wenigstens eines der Rückschlagventile (8, 9) eine bewegliche flexible Membran umfasst.

4. Einheit nach einem der vorstehenden Ansprüche, **gekennzeichnet durch** Umfassen eines Pumpelements (17), das in Reihe zwischen der Saugverzweigung (3) und der Zufiihrungsverzweigung (5) angeordnet ist.

5. Einheit nach einem der vorstehenden Ansprüche, **dadurch gekennzeichnet, dass** wenigstens der Anschluss (2), die Saugverzweigung (3) und die Zuführungsverzweigung (5) unentfernbar verbunden sind.

6. Einheit nach einem der vorstehenden Ansprüche, **dadurch gekennzeichnet, dass** sie aus biomedizinischen Polymer-Materialien hergestellt ist.

7. Einheit nach einem der vorstehenden Ansprüche, **gekennzeichnet durch** Umfassen eines Einlassanschlusses (14), der einen sterilisierenden Filter umfasst und in Reihe zwischen der Saugverzweigung (3) und der Zuführungsverzweigung (5) angeordnet ist.

8. Verfahren zum Einsetzen einer Einheit nach Anspruch 7, **gekennzeichnet durch** das vollständige Füllen der Einheit mit einer Flüssigkeit, die **durch** den Einlassanschluss (14) injiziert wird.

9. Spüleinheit nach einem der Ansprüche 1 bis 7, **gekennzeichnet durch** Umfassen eines faltbaren Elements (11), das in einer Weise faltbar ist, die **durch** Berührung und/oder visuell wahrnehmbar ist.

10. Spüleinheit nach Anspruch 9, **dadurch gekennzeichnet, dass** das faltbare Element (11) den Behälter umfasst.

11. Spüleinheit nach einem der Ansprüche 9 und 10, **dadurch gekennzeichnet, dass** das faltbare Element (11) sich faltet, wenn der Innendruck weniger als -250 mmHg beträgt.

## Revendications

1. Unité de rinçage et de remplissage de cathéter comprenant un connecteur (2) pour le raccordement à un cathéter ; un clapet de non retour (8) raccordé fluidiquement audit connecteur (2) ; une ramification d'aspiration (3) raccordée audit clapet de non retour (8) ; un filtre (15) en aval de ladite ramification d'aspiration (3) ; une ramification de distribution (5) en aval de ladite ramification d'aspiration (3) et un clapet de non retour supplémentaire (9) raccordant ladite ramification de distribution (5) audit connecteur (2) ; ladite ramification de distribution (5) comprenant un réservoir (11) ayant une paroi latérale transparente ; et ledit filtre (15) étant à l'intérieur dudit réservoir (11), l'unité étant **caractérisée en ce que** ledit clapet de non retour supplémentaire (9) est en aval dudit réservoir (11) de sorte qu'un fluide s'écoule à partir dudit réservoir (11) en passant par ledit clapet de non retour supplémentaire (9).

2. Unité selon la revendication 1, **caractérisée en ce que** ledit filtre (15) n'est pas amovible dudit réservoir (15).

3. Unité selon l'une ou l'autre des revendications précédentes, **caractérisée en ce qu'**au moins l'un desdits clapets de non retour (8, 9) comprend une membrane souple flottante.

4. Unité selon l'une quelconque des revendications précédentes, **caractérisée en ce qu'**elle comprend un élément de pompage (17) positionné en série entre ladite ramification d'aspiration (3) et ladite ramification de distribution (5).

5. Unité selon l'une quelconque des revendications précédentes, **caractérisée en ce qu'**au moins ledit connecteur (2), ladite ramification d'aspiration (3) et ladite ramification de distribution (5) sont raccordés de manière non amovible.

6. Unité selon l'une quelconque des revendications précédentes, **caractérisée en ce qu'**elle est réalisée avec des matériaux polymères biomédicaux.

7. Unité selon l'une quelconque des revendications précédentes, **caractérisée en ce qu'**elle comprend un orifice d'entrée (14) comprenant un filtre de stérilisation et positionné en série entre ladite ramification d'aspiration (3) et ladite ramification de distribution (5).

8. Procédé pour utiliser une unité selon la revendication 7, **caractérisé par** l'étape consistant à remplir complètement ladite unité avec un liquide injecté par ledit orifice d'entrée (14).

9. Unité de rinçage selon l'une quelconque des revendications 1 à 7, **caractérisée en ce qu'**elle comprend un élément repliable (11), repliable d'une manière perceptible au toucher et/ou visuellement.

10. Unité de rinçage selon la revendication 9, **caractérisée en ce que** ledit élément repliable (11) comprend ledit réservoir.

11. Unité de rinçage selon l'une ou l'autre des revendications 9 et 10, **caractérisée en ce que** ledit élément repliable (11) se replie lorsque la pression à l'intérieur est inférieure à - 250 mmHg.
